# EUROPEAN PATENT SPECIFICATION

(11) **EP 2 544 736 B1**
(45) Date of publication and mention of the grant of the patent: **09.05.2018**
(21) Application number: 11755717.3
(22) Date of filing: 03.03.2011
(51) Int. Cl.: A61M 5/168, G08B 29/02

(54) **A COMBINATION OF A PORTABLE MONITORING DEVICE AND A MEDICAL DEVICE AND A METHOD OF MONITORING A MEDICAL DEVICE**
KOMBINATION AUS EINER TRAGBAREN ÜBERWACHUNGSVORRICHTUNG UND EINER MEDIZINISCHEN VORRICHTUNG SOWIE VERFAHREN ZUR ÜBERWACHUNG EINER MEDIZINISCHEN VORRICHTUNG
COMBINAISON D'UN DISPOSITIF DE SURVEILLANCE PORTABLE ET D'UN DISPOSITIF MÉDICAL ET MÉTHODE DE SURVEILLANCE D'UN DISPOSITIF MÉDICAL

(30) Priority: 10.03.2010 US 312334 P; 28.04.2010 DK 201070174; 26.05.2010 DK 201070216; 10.06.2010 DK 201070255; 21.06.2010 DK 201070279; 30.08.2010 DK 201000760
(43) Date of publication of application: 16.01.2013
(73) Proprietor: Dripmate A/S, 2920 Charlottenlund (DK)
(72) Inventor: MERNOE, Morten, DK-2920 Charlottenlund (DK); THING, Morten, DK-3060 Espegaerde (DK)
(74) Representative: Orsnes, Henrik Egede
(86) International application number: PCT/DK2011/050065
(87) International publication number: WO 2011/113435

(56) References cited:
- EP-A1- 2 140 893
- WO-A1-2009/032402
- WO-A2-02/49509
- WO-A2-2007/081947
- US-A1- 2007 258 395
- US-A1- 2008 009 787
- US-A1- 2008 300 572
- US-A1- 2009 069 787
- US-A1- 2009 069 787
- US-A1- 2009 076 461
- US-A1- 2009 326 722
- US-B1- 6 589 229

## Description

The present invention relates to a combination of a portable monitoring device for monitoring a medical device for use in hospitals and the like and in the form of a portable drip infusion set of the type comprising a liquid supply, a drip chamber downstream of said liquid supply for forming liquid drops and a flexible tube connecting said drip chamber with an injection needle. US 2009/076461 discloses an infusion pump and a remote monitoring device that alarms the user when connection between the infusion pump and the remote monitoring device is interrupted. In connection with such medical devices used in hospitals it is a problem that the devices are stolen which is financially harmful and can be life-threatening if the stolen device is required in an emergency.

It is therefore an object of the invention to provide a system for registering that such a medical device has been removed from a certain area with a very small time lag from removal to registration of removal and further to avoid unauthorized programming of the medical device operations without access to the monitoring device which is ideally in the hands of an authorized health care worker. The invention achieving these objects is defined in claims 1 and 13. A monitoring device comprising:
a first housing, a signal receiver for receiving a first wireless signal, a first timing device for measuring a time interval, preferably 30 seconds, an alarm device for emitting a first alarm, first electronic circuitry interconnecting said signal receiver, said first timing device and said alarm device for causing said alarm device to emit said first alarm if said first signal is not received by said signal receiver at the end of said time interval, and a first battery for providing electric energy to said signal receiver, said alarm device and said first timing device, and said medical device comprising:
a second housing, a signal emitter for emitting said first wireless signal, a second timing device for measuring said time interval, second electronic circuitry interconnecting said timing device and said signal emitter for causing said signal emitter to emit said first signal at the end of said time interval, and an electric power supply, preferably a second battery, for providing electric energy to said timing device and said signal emitter.

Hereby, if and when the medical device is displaced so far away from the intended place of use thereof that the medical device is out of the receiving range of the signal receiver of the monitoring device, then the monitoring device will emit an alarm at the end of the time interval after it has received said first signal (a so-called hand shake signal) for the last time. If this time interval is short, for instance 30 seconds, then it is possible to react quickly and have a better chance than otherwise to stop the theft. In some cases, the theft takes place while the medical device is in use, and thereby the patient supposed to be treated by the medical device may be harmed or placed in jeopardy so that even if the theft is not prevented, at least it is registered that the medical device in question is not being used in the intended manner and relevant steps can be taken to protect the patient in question.

In the invention said alarm device is adapted for emitting a second alarm and said signal emitter is adapted for emitting a second wireless signal, said monitoring device comprising third electronic circuitry adapted for registering receipt of said second signal by said signal receiver and causing said alarm device to emit said second alarm if said second signal is received by said signal receiver, and said medical device comprising fourth electronic circuitry being adapted for monitoring functional parameters of said medical device such as voltage of said power supply or second battery, any pre-determined output from said medical device or the function of said fourth electronic circuitry and further being adapted for registering deviations from pre-determined values of said functional parameters and causing said signal emitter to emit said second signal if said deviations are registered.

Thus, any malfunction of the medical device that is considered serious enough to warrant an alarm will be brought to the attention of the health care person or persons monitoring the monitoring device such that an appropriate reaction can take place to safeguard the patient in question. Said monitoring device and said medical device comprise first and second sets of electrical contacts, respectively, for mutually electrically interconnecting said monitoring device and said medical device for transmitting electronic data and/or electrical power between said monitoring device and said medical device.

Hereby the monitoring device may be used for transferring electronic data to the medical device or receive and store electronic data from the medical device as well as for receiving or transmitting electric power from or to the medical device.

In the invention said medical device comprises programming means such as a push button, a switch, a keyboard, a data transmission receiver, a data input port or the like for programming fifth electronic circuitry controlling the function of said medical device, said fifth electronic circuitry comprising blocking means for blocking said programming function of said programming means when said monitoring device is not electrically connected to said medical device through said first and second electrical contacts.

This is a valuable security measure that prevents unauthorized programming or amendment of programming of the medical device operations without access to the monitoring device which ideally is in the hands of an authorized health care worker.

In the currently preferred embodiment of the invention said monitoring device housing comprises an input port for receiving a data/electrical power plug such as a USB plug, said input port being electrically connected to said first set of electrical contacts such that data and electrical power can be transmitted to said medical device from said input port when said first and second sets of electrical contacts are interconnected.

Preferably, said monitoring device and said medical device comprise first and second sets of attachment means, respectively, for detachably attaching said monitoring device to said medical device such that said first and second sets of electrical contacts are interconnected.

In the currently preferred embodiment of the invention, said first and second alarms comprise one or more of a sensory alarm such as a vibrator, a visual alarm such as lamps of different colours and an audible alarm.

In the currently preferred embodiment of the invention, said monitoring device housing comprises attachment means such as a clip for attaching said monitoring device to the apparel of a health care provider.

Advantageously, said monitoring device housing comprises means such as push buttons or switches for manually interrupting said first and second alarms.

In the currently preferred embodiment of the invention, said first and second signals comprise a code recognizable by said first and third electronic circuitry of said monitoring device.

Hereby, a certain medical device will be "paired" with a certain monitoring device which will allow several "pairs" of medical and monitoring devices to be operational on the same premises without interfering with one another.

In the currently preferred embodiment of the invention, said code is generated by fifth electronic circuitry of said medical device and transmitted to said first and third electronic circuitry of said monitoring device through said first and second sets of electric contacts.

In the currently preferred embodiment of the invention, said electronic circuitry of the medical device comprises blocking means for blocking transmittal of data and electrical power to said medical device if said first and second electrical contacts are not in mutual electrical contact.

Hereby, it is achieved that no unauthorized programming or amendment of the authorized programming may take place unless the responsible health care take connects the monitoring device to the medical device. Furthermore, the value of the medical device, if stolen, will be diminished as it cannot be used without a corresponding monitoring device.

The medical device and said monitoring device each comprise a microprocessor arranged in a printed circuit board (PCB), said microprocessors being in electrical contact for exchanging electronic data and said medical device microprocessor controlling the operation of said medical device.

In an advantageous embodiment of the combination according to the invention the monitoring device microprocessor is pre-programmed to transmit solely one specific set of operating instructions to said medical device microprocessor.

In an advantageous embodiment of the combination according to the invention, said housing of said monitoring device is provided with a display adapted for displaying information in a visual manner.

Preferably, said display is adapted to display information generated by said monitoring device microprocessor.

Advantageously, said signal emitter may be adapted for emitting a third wireless signal comprising operational data of said medical device generated by said medical device microprocessor, and said display means may be adapted for displaying information based on said operational data transmitted by said third wireless signal and received by said signal receiver of said monitoring device.

Hereby, the responsible health care person can continuously remotely monitor the operation of the medical device.

In some cases it may be an advantage that said medical device is adapted to omit any one of said first, second and third signals. Hereby the monitoring device may be utilized solely to transmit operational data or a malfunction alarm or a hand shake or any combination of two of these functions.

In a further aspect, the invention relates to a monitoring device as set out above.

In a yet further aspect, the invention relates to a medical device as set out above.

In a final aspect, the invention relates to a method of monitoring the function of a medical device for use in hospitals and the like, and in the form of a portable drip infusion set of the type comprising a liquid supply, a drip chamber downstream of said liquid supply for forming liquid drops and a flexible tube connecting said drip chamber with an injection needle, the method comprising the steps of:
- providing a portable monitoring device able to receive wireless signals from said medical device,
- causing said medical device to send a first wireless signal at certain time intervals, preferably every 30 seconds, and
- causing said monitoring device to emit a first alarm if said monitoring device does not receive said first signal after said time interval has elapsed, and- electrically connecting said monitoring device to said medical device,-programming the functions of said medical device,- removing said monitoring device from electrical contact with said medical device, and- monitoring said monitoring device so as to register whether an alarm is emitted by said monitoring device; whereby said medical device and said monitoring device each are provided with collaborating electrical contacts for establishing direct electrical contact between said monitoring device and said medical device for transmitting electrical energy and electronic data, and wherein the functions of said medical device are programmable only when said direct electrical contact is uninterrupted. In the currently preferred embodiment of the invention, the method comprises the further steps of:
   - causing said medical device to send a second wireless signal if said medical device develops a malfunction,
   - causing said monitoring device to emit a second alarm if said monitoring device receives said second signal.

In the currently preferred embodiment of the invention, said medical device and said monitoring device each are provided with collaborating electrical contacts for establishing direct electrical contact between said monitoring device and said medical device for transmitting electrical energy and electronic data, and the functions of said medical device are programmable only when said direct electrical contact is uninterrupted.

In the currently preferred embodiment of the invention, the method comprises the steps of:
- causing said medical device to generate a code,
- transmitting said code to said monitoring device,
- incorporating said code in said first and second wireless signals.

Advantageously, said medical device comprises a first microprocessor for controlling the function of said medical device and said monitoring device comprises a second microprocessor adapted to communicate with said first microprocessor when said monitoring device is in electrical connection with said medical device and the method according to the invention comprises the steps of:
- programming said second microprocessor,
- electrically connecting said monitoring device with said medical device, and
- transmitting data from said second microprocessor to said first microprocessor.

In a further embodiment, the method according to the invention may comprise the steps of:
- generating operating data regarding the functioning of said medical device in said first microprocessor,
- transmitting said operating data to said monitoring device,
- storing said operating data in said monitoring device, and
- transmitting said operating data from said monitoring device to an electronic entity such as a PC.

Hereby the treatment of the patient may be recorded and entered into the patient's medical records.

Advantageously, said monitoring device is provided with a display, the method comprising the further step of displaying information relative to the specific programming of said monitoring device.

Advantageously, said method may comprise the further steps of causing said medical device to transmit said operating data to said monitoring device by means of a third wireless signal and displaying said operating data by means of said display.

In some cases it may be advantageous that any one of said first, second and third wireless signals is omitted. Hereby the monitoring method may be utilized solely to transmit operational data or a malfunction alarm or a hand-shake or any combination of two of these functions.

In the following, the invention will be explained more in detail in connection with the currently preferred embodiment thereof shown, solely be way of example, in the accompanying drawings, where:
Fig. 1 is a schematic, perspective view of a combination according to the invention with the monitoring device attached to a medical device in the form of a portable drip infusion device which is monitored by the monitoring device,
Fig. 2 is a schematic perspective view corresponding to Fig. 1 with the monitoring device shown separated from the medical device,
Fig. 3 is a schematic perspective view corresponding to Fig. 2 but seen from another angle,
Figs. 4-6 are schematic perspective views corresponding to Figs. 1-3 illustrating the supply of electrical power and/or data to the monitoring device and the medical device,
Figs. 7-8 are schematic enlarged perspective views of the monitoring device with the bottom covering wall removed to show the interior structure,
Fig. 9 is a schematic perspective exploded view of the medical device monitored by the monitoring device,
Figs. 10-12 are schematic, perspective views of three alternative embodiments of a monitoring device according to the invention,
Fig. 13 is a larger scale schematic perspective view of the interconnection of a monitoring device according to the invention and a personal computer (PC),
Figs. 14-15 are schematic enlarges scale perspective views of a further embodiment of monitoring device according to the invention, seen from the top and the bottom, respectively, and
Fig. 16 is a larger scale schematic perspective view of a yet further embodiment of a monitoring device according to the invention, seen from the top.

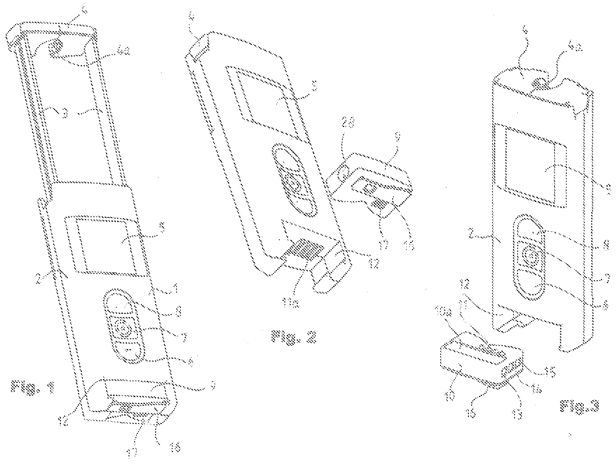

Referring now to Figs. 1-3, a medical device 1 in the form of a portable drip infusion device has a housing 2 and two telescopically displaceable arms 3 carrying a gripping mechanism 4 for gripping a not shown drip chamber and sensors 4a for sensing drops falling through said drip chamber. The device 1 has a display 5 for displaying information regarding the function of the device. Push buttons 6, 7 and 8 are provided for manually programming the function of the device, button 6 being for decreasing flow of infusion liquid, button 7 being for activating the device and button 8 being for increasing the flow of infusion liquid.

A monitoring device 9 according to the invention has a housing 10 and four electrical contact pins 11 for electrically contacting four corresponding electrical contact plates 11a arranged in a recess 12 in the bottom of housing 2, the recess being adapted for receiving the monitoring device 9.

The monitoring device 9 is provided with three LED light emitters 13, 14 and 15 adapted for emitting white, red and green light, respectively. The monitoring device is further provided with a clip 16 for attaching the monitoring device to the front of a pocket or the like of a health care worker responsible for monitoring the medical device. A space 10a on the housing 10 is provided for marking the monitoring device 9 with an erasable marker for instance to visually identify the medical device which is being monitored by the monitoring device.

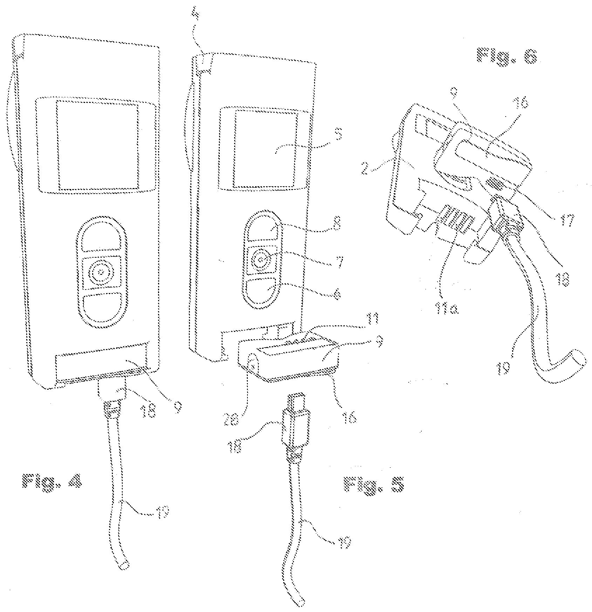

Referring now to Figs. 4-6, the monitoring device 9 is further provided with a socket for receiving a miniature USB plug 18 for supplying electrical power and/or data transmission from a cable 19 to the monitoring device 9/medical device 1 as explained more in detail in the following.

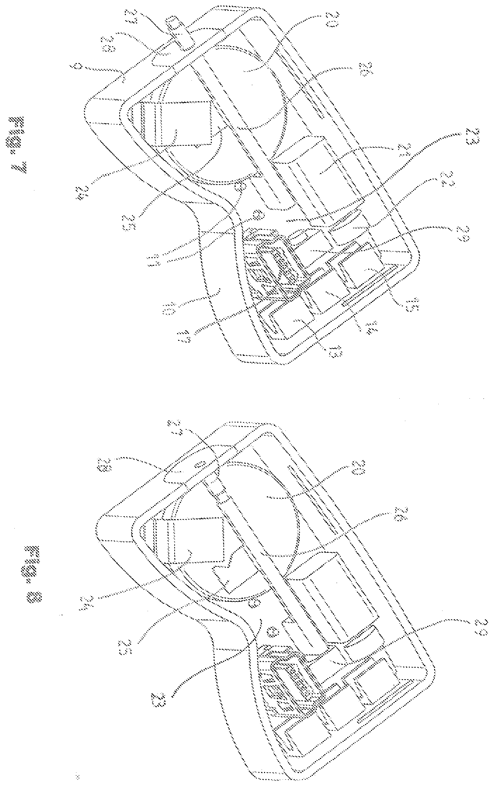

Referring now to Figs. 7 and 8 illustrating the monitoring device seen from the bottom (with the bottom wall removed) and in storage mode and in active mode, respectively, the housing 10 contains a rechargeable battery 20, a vibrator motor 21 and a vibrator 22, three LED light emitters 13-15 (green, yellow and red, respectively), a printed circuit board (PCB) 23, a battery contact plate 24 and an insulating plate 25 attached to a push rod 26. A mini USB socket 17 is electrically connected to the PCB 23. The PCB 23 comprises a not shown microprocessor.

Two electrical contacts 11 are connected to the PCB 23, the other two electrical contacts are hidden behind the socket 17 and the battery 20 and are also connected to the PCB 23. Two of the contacts 11 serve for transmitting electrical power and two contacts 11 serve for data transmission.

The push rod 26 has a telescopically displaceable portion 27 and is mounted on a displaceable plate 28. A switch 29 serves to stop the vibrator motor 21 when the plate 28 and rod 26 are displaced to the right from the position shown in Fig. 8 by finger pressure exerted by the responsible health care provider as explained more in detail in the following.

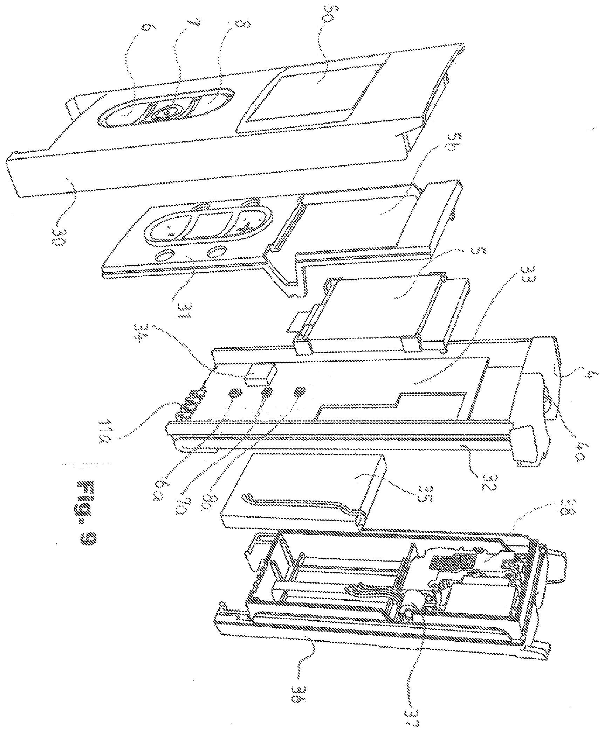

Referring now to Fig. 9 illustrating the portable drip infusion device 1 with some of the different components thereof exposed in an exploded view, a front cabinet 30 is provided with the push buttons 6-8 and is assembled with a moisture insulating rubber seal 31 unto the main body 32 of the device 1 with the display 5 received in apertures 5a and 5b in front cabinet 30 and insulating seal 31.

The main body 32 contains a printed circuit board (PCB) 33 comprising a not shown microprocessor for controlling the functions of medical device 1, generating a code for the wireless signals, programming of the functions of the medical device 1, monitoring said functions to register any malfunction of the device 1 and generating said second signal in case of a malfunction, measuring time for determining the emission of said first signal and generating said first signal and generating operational data for being displayed in the display 5.

An audible alarm 34 is mounted on the PCB 33 and switches 6a, 7a and 8a are mounted on the PCB to be activated by the push buttons 6, 7 and 8, respectively.

Four electrical contacts 11a for contacting the corresponding contacts 11 of the monitoring device 9 are also connected to the PCB 33.

A rechargeable battery 35 is mounted between the main body 32 and a rear cabinet 36 containing elements for the function of the medical device 1 such as a motor 37 and a gear box 38 for regulating the flow of infusion liquid.

In use, the monitoring device shown in Fig. 7 is readied for its first time use by pressing the portion 27 of the rod 26 such that the rod is displaced to the right in Fig. 7 whereby the insulating plate is displaced to the position shown in Fig. 8 where it does not prevent contact between the battery 20 and the battery contact plate whereby power is now supplied to the PCB 23. Hereby, it is avoided that the battery 20 is discharged during storage prior to first use of the monitoring device 9.

The monitoring device 9 is inserted in the recess 12 in the medical device 1 such that the contacts 11 and 11a are electrically connected. The mini USB plug 18 may, if necessary, be inserted in the socket 17 to recharge the batteries 20 and 35 and perhaps transmit electronic data to the microprocessor of the medical device 1. The battery 35 of the medical device can only be recharged through the corresponding monitoring device's USB plug 18.

The rate of flow of the infusion device 1 is set by means of the buttons 6 and 8. A code is generated by the microprocessor incorporated in the PCB 33 of the medical device 1 and transmitted to and stored in the microprocessor incorporated in the PCB 23 of the monitoring device 9 through the contacts 11 and 11a. This allows several different medical devices to be "paired" with each its own monitoring device in the same hospital.

The medical device 1 is activated by pressing the button 7 and the monitoring device is removed from the recess 12 and for instance clipped to the breast pocket of the responsible health care provider.

The green LED 13 blinks as long as the medical device 1 is functioning correctly and the battery 35 thereof has sufficient power.

Every 30 seconds the signal emitter incorporated in the PCB 33 of the medical device 1 sends a "hand shake" signal with the code incorporated, and as long as the medical device is within receiving range of the signal receiver incorporated in the PCB 23 of the monitoring device and therefore the signal is received by the signal receiver, the monitoring device will not react. However, if the hand shake signal is not received after the elapse of 30 seconds after the last hand shake signal was received, the vibrator 22 of the monitoring device will be activated and the yellow LED 14 will start blinking which will indicate that the medical device has been moved out of range of the monitor device's signal receiver.

The health care provider can stop the vibrator by pushing on the push button 28 thereby activating the vibrator interruption switch, but the yellow LED 14 will continue blinking until a new hand shake signal is received.

The microprocessor incorporated in the PCB 33 of the medical device monitors the correct operation of the medical device and the condition of the battery 35. If a malfunction is detected, an alarm signal will be transmitted by the signal transmitter incorporated in the PCB 33. When this alarm signal is received by the signal receiver incorporated in the PCB 23, the vibrator 22 will be activated, and the red LED 15 will start blinking. The vibrator can be deactivated by pushing the button 28, but the red LED 15 will continue blinking until the monitoring device is inserted into the recess 12 of the medical device.

As a further embodiment of the combination according to the invention as an alternative to the medical device 1, for instance a drip infusion set, being programmed with specific functional parameters, in the case of drip infusion sets, drop size and flow rate by the user (nurse) when setting up the treatment of a patient this programming step by the nurse can be eliminated.

In some hospital or clinic environments it is preferred only to use one specific drop set or drip chamber and therefore only utilize one specific drop size. Then there is no need for the user to programme drop size into the medical device, and eliminating programming of drop size also eliminates the risk of programming the wrong drop size into the device.

In that case it would be an advantage to the user if the monitoring device used is able to programme the device with a specific drop size even though the medical device is adapted to be programmed for several different drop sizes.

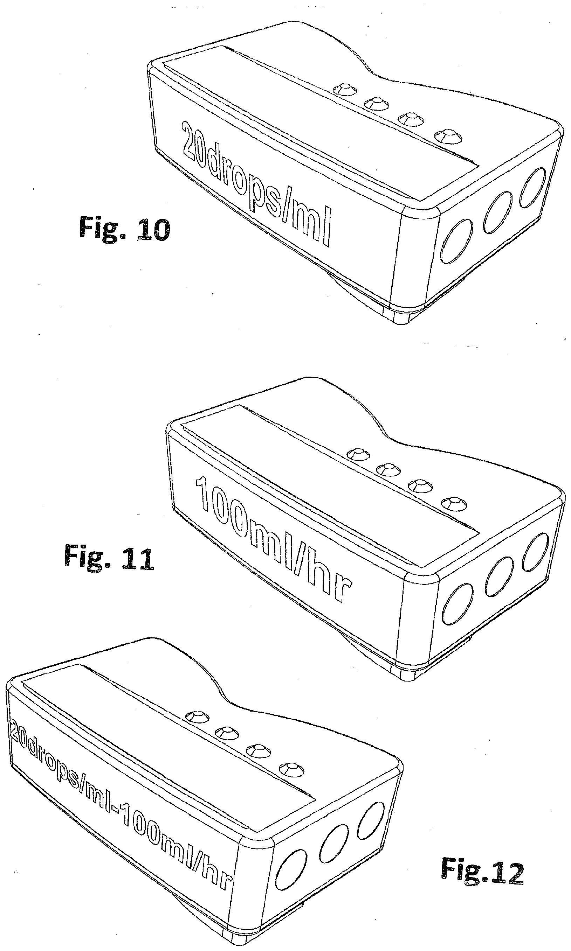

To achieve this advantage the microprocessor of the PCB 23 of the monitoring device is factory programmed to a specific drop size and marked (see Fig. 10) or colour coded to indicate to the user whether the monitoring device will programme the medical device to: 10, 15, 20, 30, 40, 50 or 60 drop/ml (or any other suitable number).

Of course, this alternative also applies to other programmable medical devices for use in a combination according to the invention.

In some environments only one type of infusion is performed and therefore only one flow rate needs to be programmed, and in these cases it would be an advantage to the user if the monitoring device used is able to programme the medical device with a specific flow rate even though the medical device is adapted to be programmed for several different flow rates.

To achieve this advantage the monitoring device microprocessor is factory programmed to a specific flow rate and marked (see Fig. 11) or colour coded to indicate to the user whether the monitoring device will programme the medical device to: 10, 50, 100, 150, 200 or 250 ml/hr (or any other suitable number).

Alternatively, the monitoring device microprocessor can be factory programmed with both a specific drop size and a specific flow rate in cases where the user always uses the same specific drop set and only at a specific flow rate (see Fig. 12).

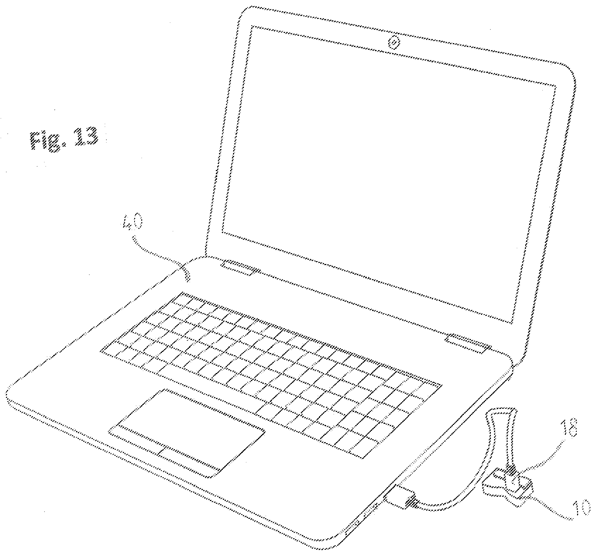

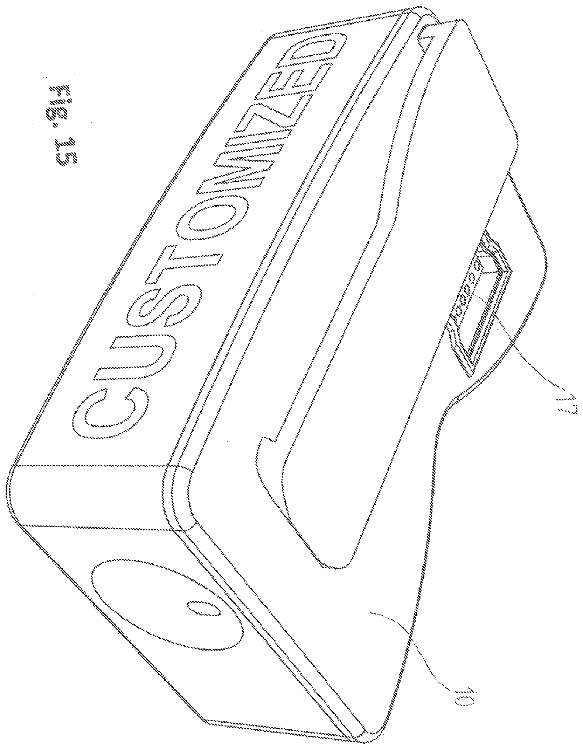

Reference is now made to Fig. 13-15. In some hospital or clinic environments, the doctor in charge of the treatment of a patient could be interested in programming the drop size and flow rate himself into the microprocessor of the monitoring device to take the responsibility for programming the medical device off the nurse's shoulders.

In this version of the monitoring device it can be connected to a PC 40 by inserting the USB plug 18 in the USB socket 17 (see Fig. 13), and the doctor can program the monitoring device's microprocessor and when the monitoring device is electrically connected to the medical device, the medical device's microprocessor, so that the medical device will work with a specific drop size and flow rate. Other functional parameters for the medical device can also be programmed by a doctor (or nurse) in this manner.

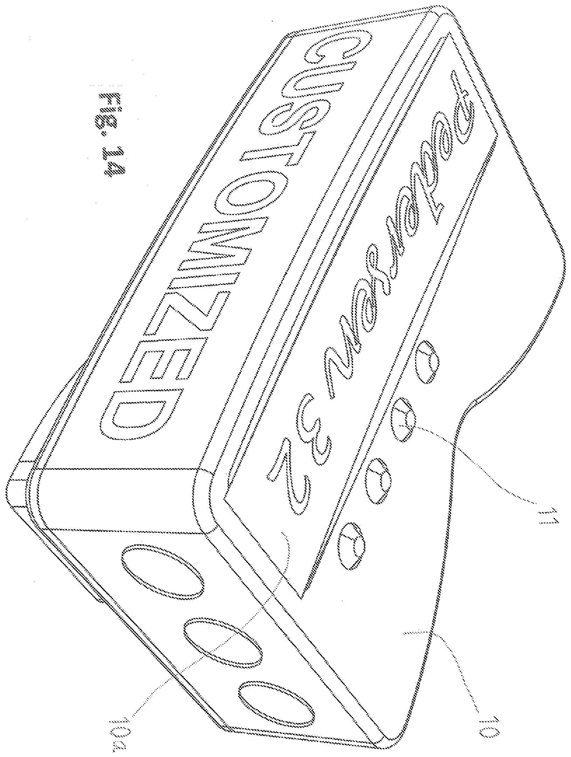

To make sure that a specific, individually programmed monitoring device is used for the treatment of the corresponding specific patient the doctor can write the patients name in the text field 10a (see Figs. 14-15).

On the front of the monitoring device, the text "CUSTOMIZED" is applied to indicate that the monitoring device is specific and cannot be used for any but an intended medical device.

The medical device 1 can only be stopped and started with the monitoring device 9 inserted in the medical device and in electrical contact therewith. In hospitals and clinics where electronic journals are used it could be an advantage if the monitoring device is able to record and store data describing the treatment performed by the medical device. Then the doctor can connect the monitoring device via the USB connection to a PC and read the treatment data stored in the monitoring device and save them in the patients electronic journal.

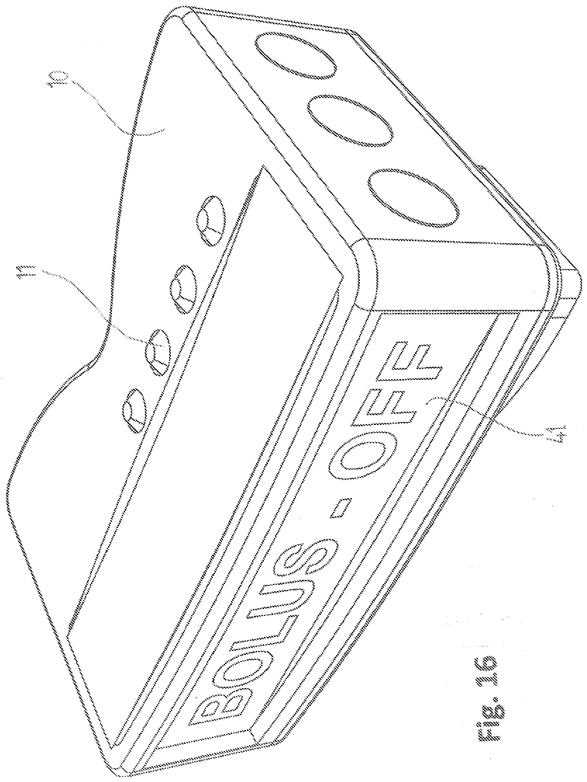

Referring now to Fig. 16, in case the monitoring device 9 is programmed, for instance by a PC 40, it could be an advantage if a display 41 is provided in the housing 10 of the monitoring device. This display shows the data which the monitoring device has been programmed with. This will allow the medical device to perform more sophisticated treatments than just a fixed flow rate. For instance specify duration of the infusion, variation over time in flow rate, intermediated flow rates, Bolus option (for instance by pressing a specific key on the device) and bolus lock period.

The display can then show the specific features programmed, for instance as remaining time if a time limited treatment is programmed, bolus off/on if a bolus and a bolus lock period is programmed or a pause if an intermediate treatment has been programmed.

In Fig. 16, the display 41 displays the information "BOLUS-OFF" indicating that the bolus is in lock period.

In a further embodiment, the microprocessor incorporated in the PCB 33 of the medical device registers various data regarding the operation of the medical device and this data is communicated to the signal transmitter that transmits this data as a third signal to the monitoring device to be displayed by the display 41 such that the operation of the medical device can be monitored by a doctor or a nurse.

## Claims

1. A combination of a portable monitoring device (9) for monitoring a medical device (1) for use in hospitals and the like and in the form of a portable drip infusion set of the type comprising a liquid supply, a drip chamber downstream of said liquid supply for forming liquid drops and a flexible tube connecting said drip chamber with an injection needle, and the medical device (1)
- said monitoring device (9) comprising:
a first housing (10), a signal receiver for receiving a first wireless signal, a first timing device for measuring a first time interval, preferably 30 seconds, an alarm device for emitting a first alarm, first electronic circuitry interconnecting said signal receiver, said first timing device and said alarm device for causing said alarm device to emit said first alarm if said first signal is not received by said signal receiver at the end of said first time interval, and a first battery for providing electric energy to said signal receiver, said alarm device and said timing device,
- said medical device (1) comprising:
a second housing (2), a signal emitter for emitting said first wireless signal, a second timing device for measuring said first time interval, second electronic circuitry interconnecting said timing device and said signal emitter for causing said signal emitter to emit said first signal at the end of said first time interval, and an electric power supply, preferably a second battery, for providing electric energy to said timing device and said signal emitter; whereby
• said alarm device is adapted for emitting a second alarm and said signal emitter is adapted for emitting a second wireless signal, said monitoring device (9) comprising third electronic circuitry adapted for registering receipt of said second signal by said signal receiver and causing said alarm device to emit said second alarm if said second signal is received by said signal receiver, and said medical device (1) comprising fourth electronic circuitry being adapted for monitoring functional parameters of said medical device such as voltage of said power supply or second battery, any pre-determined output from said medical device (1) or the function of said fourth electronic circuitry and further being adapted for registering deviations from pre-determined values of said functional parameters and causing said signal emitter to emit said second signal if said deviations are registered; and
• said monitoring device (9) and said medical device (1) comprise first and second sets of electrical contacts (11, 11a), respectively, for mutually electrically interconnecting said monitoring device (9) and said medical device (1) for transmitting electronic data and/or electrical power between said monitoring device (9) and said medical device (1), and
• said medical device (1) comprises programming means such as a push button, a switch, a keyboard, a data transmission receiver, a data input port or the like, for programming fifth electronic circuitry controlling the function of said medical device (1), **characterized in that** said fifth electronic circuitry comprising blocking means for blocking said programming function of said programming means when said monitoring device (9) is not electrically connected to said medical device (1) through said first and second electrical contacts.

2. A combination according to claim 1, wherein said monitoring device housing (10) comprises an input port for receiving a data/electrical power plug such as a USB plug, said input port being electrically connected to said first set of electrical contacts (11) such that data and electrical power can be transmitted to said medical device (1) from said input port when said first and second sets of electrical contacts (11, 11a) are interconnected.

3. A combination according to claim 2 or 3, wherein said monitoring device (9) and said medical device (1) comprise first and second sets of attachment means, respectively, for detachably attaching said monitoring device (9) to said medical device (1) such that said first and second sets of electrical contacts (11, 11a) are interconnected.

4. A combination according to any of the preceding claims, wherein said first and second alarms comprise one or more of a sensory alarm such as a vibrator, a visual alarm such as lamps of different colours and an audible alarm.

5. A combination according to any of the preceding claims, wherein said monitoring device housing (10) comprises attachment means such as a clip (16) for attaching said monitoring device (9) to the apparel of a health care provider.

6. A combination according to any of the preceding claims, wherein said monitoring device housing (10) comprises means such as push buttons or switches for manually interrupting said first and second alarms.

7. A combination according to any of the claims 1- 6, wherein said first and second signals comprise a code recognizable by said first and third electronic circuitry of said monitoring device (9).

8. A combination according to claim 10, wherein said code is generated by fifth electronic circuitry of said medical device (1) and transmitted to said first and third electronic circuitry of said monitoring device (9) through said first and second sets of electric contacts (11, 11a).

9. A combination according to any of the claims 5-18, wherein said electronic circuitry of the medical device (1) comprises blocking means for blocking transmittal of data and electrical power to said medical device (1) if said first and second electrical contacts (11, 11a) are not in mutual electrical contact.

10. A combination according to any of the preceding claims, wherein said medical device (1) and said monitoring device (9) each comprise a microprocessor arranged in a printed circuit board (PCB) (33, 23), said microprocessors being in electrical contact for exchanging electronic data and said medical device microprocessor controlling the operation of said medical device (1).

11. A combination according to claim 10, wherein said monitoring device microprocessor is pre-programmed to transmit solely one specific set of operating instructions to said medical device microprocessor.

12. A combination according to any of the preceding claims, wherein said housing (10) of said monitoring device (9) is provided with a display (41) adapted for displaying information in a visual manner.

13. A method of monitoring the function of a medical device (1) for use in hospitals and the like, and in the form of a portable drip infusion set of the type comprising a liquid supply, a drip chamber downstream of said liquid supply for forming liquid drops and a flexible tube connecting said drip chamber with an injection needle, the method comprising the steps of:
- providing a combination of a portable monitoring device (9) able to receive wireless signals from said medical device (1), and said medical device (1), the combination according to any of the preceding claims
- causing said medical device (1) to send a first wireless signal at certain time intervals, preferably every 30 seconds, and
- causing said monitoring device (9) to emit a first alarm if said monitoring device (9) does not receive said first signal after said time interval has elapsed, and
- electrically connecting said monitoring device (9) to said medical device (1),
- programming the functions of said medical device (1),
- removing said monitoring device (9) from electrical contact with said medical device (1), and
- monitoring said monitoring device (9) so as to register whether an alarm is emitted by said monitoring device (9); **characterized in that** said medical device (1) and said monitoring device (9) each are provided with collaborating electrical contacts (11, 11a) for establishing direct electrical contact between said monitoring device (9) and said medical device (1) for transmitting electrical energy and electronic data, and wherein the functions of said medical device (1) are programmable only when said direct electrical contact is uninterrupted.

## Patentansprüche

1. Kombination einer tragbaren Überwachungsvorrichtung (9) zur Überwachung einer medizinischen Vorrichtung (1) zur Verwendung in Krankenhäusern und dergleichen und in Form eines tragbaren Tropfinfusionssets der Bauart, die eine Flüssigkeitszufuhr, eine dieser Flüssigkeitszufuhr nachgelagerte Tropfkammer zur Bildung von Flüssigkeitstropfen und einen flexiblen Schlauch, der die Tropfkammer mit einer Injektionsnadel verbindet, und die medizinische Vorrichtung (1) umfasst - die Überwachungsvorrichtung (9) umfasst:
ein erstes Gehäuse (10),
einen Signalempfänger zum Empfangen eines ersten drahtlosen Signals, eine erste Zeitmessvorrichtung zum Messen eines ersten Zeitintervalls, vorzugsweise 30 Sekunden, eine Alarmvorrichtung zum Aussenden eines ersten Alarms, eine erste elektronische Schaltung die diesen Signalempfänger verbindet, die erste Zeitmessvorrichtung und die Alarmeinrichtung um die Alarmeinrichtung zu veranlassen, den ersten Alarm auszulösen, wenn das erste Signal am Ende des ersten Zeitintervalls nicht vom Signalempfänger empfangen wird, und eine erste Batterie zum Liefern von elektrischer Energie an den Signalempfänger, die Alarmvorrichtung und die Zeitmessvorrichtung,
- die medizinische Vorrichtung (1) umfasst:
ein zweites Gehäuse (2),
einen Signalemitter zum Aussenden des ersten drahtlosen Signals, eine zweite Zeitmessvorrichtung zum Messen des ersten Zeitintervalls, eine zweite elektronische Schaltung, die die Zeitmessvorrichtung und den Signalemitter miteinander verbindet, um zu bewirken, dass der Signalemitter das erste Signal am Ende des ersten Zeitintervalls aussendet, und eine elektrische Stromversorgung, vorzugsweise eine zweite Batterie, zum Liefern von elektrischer Energie an die Zeitmessvorrichtung und den Signalemitter; wobei
• die Alarmvorrichtung zum Aussenden eines zweiten Alarms ausgelegt ist und der Signalemitter zum Aussenden eines zweiten drahtlosen Signals ausgelegt ist, wobei die Überwachungsvorrichtung (9) eine dritte elektronische Schaltung zum Registrieren des Empfangs des zweiten Signals durch den Signalempfänger umfasst und veranlasst, dass die Alarmvorrichtung den zweiten Alarm auslöst, wenn das zweite Signal von dem Signalempfänger empfangen wird, und wobei die medizinische Vorrichtung (1) eine vierte elektronische Schaltung umfasst, die zum Überwachen von Funktionsparametern der medizinischen Vorrichtung wie Spannung der Stromversorgung oder zweiter Batterie, jeder vorbestimmten Ausgabe der medizinischen Vorrichtung (1) oder der Funktion der vierten elektronischen Schaltung ausgelegt ist und ferner ausgelegt ist, um Abweichungen von vorbestimmten Werten der funktionalen Parameter zu registrieren und zu bewirken, dass der Signalemitter das zweite Signal aussendet, wenn die Abweichungen registriert sind; und
• die Überwachungsvorrichtung (9) und die medizinische Vorrichtung (1) jeweils erste und zweite Sätze elektrischer Kontakte (11, 11a) umfassen, um die Überwachungsvorrichtung (9) und die medizinische Vorrichtung (1) zur Übertragung von elektronischen Daten und/oder elektrischer Energie zwischen der Überwachungsvorrichtung (9) und der medizinischen Vorrichtung (1) elektrisch miteinander zu verbinden, und
• die medizinische Vorrichtung (1) Programmiereinrichtungen wie einen Druckknopf, einen Schalter, eine Tastatur, einen Datenübertragungsempfänger, einen Dateneingabeport oder dergleichen zum Programmieren einer fünften elektronischen Schaltung umfasst, die die Funktion der medizinischen Vorrichtung (1) steuert, dadurch charakterisiert, dass die fünfte elektronische Schaltung eine Blockiereinrichtung zum Blockieren der Programmierfunktion der Programmiereinrichtung umfasst, wenn die Überwachungseinrichtung (9) nicht über die ersten und zweiten elektrischen Kontakte mit der medizinischen Vorrichtung (1) elektrisch verbunden ist.

2. Kombination nach Anspruch 1, wobei das Gehäuse der Überwachungsvorrichtung (10) einen Eingangsport zum Empfangen eines Daten-/elektrischen Netzsteckers, wie etwa eines USB-Steckers, umfasst, wobei der Eingangsport mit dem ersten Satz elektrischer Kontakte (11) elektrisch verbunden ist, so dass Daten und elektrische Energie zu der medizinischen Vorrichtung (1) übertragen werden können, wenn der erste und zweite Satz elektrischer Kontakte (11, 11a) miteinander verbunden sind.

3. Kombination nach Anspruch 2 oder 3, wobei die Überwachungsvorrichtung (9) und die medizinische Vorrichtung (1) jeweils erste und zweite Sätze von Befestigungseinrichtungen umfassen, um die Überwachungsvorrichtung (9) an der medizinischen Vorrichtung(1) abnehmbar so zu befestigen, dass der erste und der zweite Satz elektrischer Kontakte (11, 11a) miteinander verbunden sind.

4. Kombination nach einem der vorhergehenden Ansprüche, wobei der erste und der zweite Alarm einen oder mehrere sensorische/n Alarm/e, wie ein Vibrieren, einen optischen Alarm wie beispielsweise Lampen unterschiedlicher Farben und einen hörbaren Alarm umfassen.

5. Kombination nach einem der vorhergehenden Ansprüche, wobei das Gehäuse der Überwachungsvorrichtung (10) Befestigungseinrichtungen wie eine Klammer (16) zum Befestigen der Überwachungsvorrichtung (9) an der Kleidung eines Gesundheitsdienstleisters umfasst.

6. Kombination nach einem der vorhergehenden Ansprüche, wobei das Gehäuse der Überwachungsvorrichtung (10) Einrichtungen wie Drucktasten oder Schalter zum manuellen Unterbrechen des ersten und des zweiten Alarms umfasst.

7. Kombination nach einem der Ansprüche 1-6, wobei das erste und zweite Signal einen Code umfassen, der durch die erste und dritte elektronische Schaltung der Überwachungsvorrichtung (9) erkennbar ist.

8. Kombination nach Anspruch 7, wobei der Code durch eine fünfte elektronische Schaltung der medizinischen Vorrichtung (1) erzeugt wird und über die ersten und zweiten Sätze von elektrischen Kontakten (11, 11a) an die erste und dritte elektronische Schaltung der Überwachungsvorrichtung (9) übertragen wird.

9. Kombination nach einem der Ansprüche 8, wobei die elektronische Schaltung der medizinischen Vorrichtung (1) eine Blockiereinrichtung zum Blockieren der Übertragung von Daten und elektrischer Energie zu der medizinischen Vorrichtung (1) umfasst, wenn der erste und der zweite elektrische Kontakt (11, 11a) nicht in gegenseitigem elektrischem Kontakt stehen.

10. Kombination nach einem der vorhergehenden Ansprüche, wobei die medizinische Vorrichtung (1) und die Überwachungsvorrichtung (9) jeweils einen in einer Leiterplatte (PCB) (33, 23) angeordneten Mikroprozessor umfassen, wobei die Mikroprozessoren zum Austausch elektronischer Daten in elektrischem Kontakt stehen und der Mikroprozessor den Betrieb der medizinischen Vorrichtung (1) steuert.

11. Kombination nach Anspruch 10, wobei der Mikroprozessor der Überwachungsvorrichtung vorprogrammiert ist, um nur einen bestimmten Satz Bedienungsanweisungen an den Mikroprozessor der medizinischen Vorrichtung zu übertragen.

12. Kombination nach einem der vorhergehenden Ansprüche, wobei das Gehäuse (10) der Überwachungsvorrichtung (9) mit einer Anzeige (41) versehen ist, die zur visuellen Anzeige von Informationen ausgelegt ist.

13. Ein Verfahren zur Überwachung der Funktion einer medizinischen Vorrichtung (1) zur Verwendung in Krankenhäusern und dergleichen und in Form eines tragbaren Tropfinfusionssets der Bauart, die eine Flüssigkeitszufuhr, eine dieser Flüssigkeitszufuhr nachgelagerte Tropfkammer zur Bildung von Flüssigkeitstropfen und einen flexiblen Schlauch, der die Tropfkammer mit einer Injektionsnadel verbindet, umfasst, wobei das Verfahren die Schritte umfasst:
- Bereitstellen einer Kombination einer tragbaren Überwachungsvorrichtung (9), die drahtlose Signale von der medizinischen Vorrichtung (1) empfangen kann, und dieser medizinischen Vorrichtung (1), die Kombination nach einem der vorhergehenden Ansprüche
- die medizinische Vorrichtung (1) zu veranlassen ein erstes drahtloses Signal in bestimmten Zeitintervallen zu senden, vorzugsweise alle 30 Sekunden, und
- die Überwachungsvorrichtung (9) zu veranlassen einen ersten Alarm abzugeben, wenn die Überwachungsvorrichtung (9) das erste Signal nicht empfängt, nachdem das Zeitintervall verstrichen ist, und
- elektrisches Verbinden der Überwachungsvorrichtung (10) mit der medizinischen Vorrichtung (1),
- Programmieren der Funktionen der medizinischen Vorrichtung (1),
- Entfernen der Überwachungsvorrichtung (9) vom elektrischen Kontakt mit der medizinischen Vorrichtung (1), und
- Überwachen der Überwachungsvorrichtung (9), um zu erfassen, ob ein Alarm von der Überwachungsvorrichtung (9) abgegeben wird;
charakterisiert dadurch, dass die medizinische Vorrichtung (1) und die Überwachungseinheit (9) jeweils mit zusammenwirkenden elektrischen Kontakten (11, 11a) versorgt sind um einen direkten elektrischen Kontakt zwischen der Überwachungseinheit (9) und der medizinischen Vorrichtung (1) aufzubauen, um elektrische Energie und elektronische Daten zu übertragen, und wobei die Funktionen der medizinischen Vorrichtung (1) nur programmierbar sind, wenn dieser direkte elektronische Kontakt ununterbrochen ist.

## Revendications

1. Combinaison d'un dispositif de surveillance portable (9) pour surveiller un dispositif médical (1) pour l'utilisation dans des hôpitaux et analogues et sous la forme d'un ensemble de perfusion goutte à goutte portable du type comprenant une alimentation en liquide, une chambre d'écoulement en aval de ladite alimentation en liquide pour former des gouttes de liquide et un tube flexible reliant ladite chambre d'écoulement avec une aiguille d'injection, et le dispositif médical (1)
- ledit dispositif de surveillance (9) comprenant:
un premier boîtier (10), un récepteur de signal pour recevoir un premier signal sans fil, un premier dispositif de chronométrage pour mesurer premier intervalle de temps, de préférence 30 secondes, un dispositif d'alarme pour émettre une première alarme, premier circuit électronique reliant ledit récepteur de signal, ledit premier dispositif de chronométrage et ledit dispositif d'alarme pour faire émettre par ledit dispositif d'alarme ladite première alarme si ledit premier signal n'est pas reçu par ledit récepteur de signal à la fin dudit premier intervalle de temps, et une première batterie pour fournir énergie électrique audit récepteur de signal, audit dispositif d'alarme et audit dispositif de chronométrage,
- ledit dispositif médical (1) comprenant:
un deuxième boîtier (2), un émetteur de signaux pour émettre ledit premier signal sans fil, un second dispositif de chronométrage pour mesurer ledit premier intervalle de temps, deuxième circuit électronique reliant ledit dispositif de chronométrage et ledit émetteur de signaux pour faire émettre par ledit émetteur de signaux ledit premier signal à la fin dudit premier intervalle de temps, et une alimentation électrique, de préférence une deuxième batterie, pour fournir énergie électrique audit dispositif de chronométrage et audit émetteur de signaux;
dans lequel
• ledit dispositif d'alarme est adapté pour émettre une deuxième alarme et ledit émetteur de signaux est adapté pour émettre un deuxième signal sans fil, ledit dispositif de surveillance (9) comprenant troisième circuit électronique adaptée pour registrer réception dudit deuxième signal par ledit récepteur de signal et faire émettre par ledit dispositif d'alarme ladite deuxième alarme si ledit deuxième signal est reçu par ledit récepteur de signal, et ledit dispositif médical (1) comprenant quatrième circuit électronique adaptée pour monitorer des paramètres de fonctionnement dudit dispositif médical tels que la tension de ladite alimentation ou de ladite deuxième batterie, toute sortie prédéterminée dudit dispositif médical (1) ou la fonction dudit quatrième circuit électronique et étant en outre adaptée pour registrer déviations par rapport à des valeurs prédéterminées desdits paramètres de fonctionnement et faire émettre par ledit émetteur de signaux ledit deuxième signal si lesdits déviations sont enregistrées; et
• ledit dispositif de surveillance (9) et ledit dispositif médical (1) comprennent premier et deuxième ensembles de contacts électriques (11, 11a), respectivement, pour interconnecter électriquement ledit dispositif de surveillance (9) et ledit dispositif médical (1) pour transmettre des données électroniques et/ou énergie électrique entre ledit dispositif de surveillance (9) et ledit dispositif médical (1), et
• ledit dispositif médical (1) comprend des moyens de programmation tels qu'un bouton-poussoir, un commutateur, un clavier, un récepteur de transmission de données, une porte d'entrée de données ou analogue pour programmer cinquième circuit électronique contrôlant la fonction dudit dispositif médical (1),
**caractérisé en ce que**
ledit cinquième circuit électronique comprend des moyens de blocage pour bloquer ladite fonction de programmation desdits moyens de programmation lorsque ledit dispositif de surveillance (9) n'est pas connecté électriquement audit dispositif médical (1) par lesdits premier et deuxième contacts électriques.

2. Combinaison selon la revendication 1, dans laquelle ledit boîtier (10) de dispositif de surveillance comprend une porte d'entrée pour recevoir une prise des données / énergie électrique telle qu'une prise USB, ladite porte d'entrée étant connectée électriquement audit premier ensemble de contacts électriques (11) de sorte que les données et l'énergie électrique peuvent être transmises audit dispositif médical (1) à partir de ladite porte d'entrée lorsque lesdits premier et deuxième ensembles de contacts électriques (11, 11a) sont interconnectés.

3. Combinaison selon la revendication 2 ou 3, dans laquelle ledit dispositif de surveillance (9) et ledit dispositif médical (1) comprennent premier et deuxième ensembles de moyens de fixation, respectivement, pour attacher de manière détachable ledit dispositif de surveillance (9) audit dispositif médical (1) de sorte que lesdits premier et deuxième ensembles de contacts électriques (11, 11a) sont interconnectés.

4. Combinaison selon l'une quelconque des revendications précédentes, dans laquelle lesdites première et deuxième alarmes comprennent une ou plusieurs alarme sensorielle telle qu'un vibrateur, une alarme visuelle telle que des lampes de différentes couleurs et une alarme sonore.

5. Combinaison selon l'une quelconque des revendications précédentes, dans laquelle ledit boîtier (10) de dispositif de surveillance comprend des moyens de fixation tel qu'un clip (16) pour attacher ledit dispositif de surveillance (9) aux vêtements d'un fournisseur de soins de santé.

6. Combinaison selon l'une quelconque des revendications précédentes, dans laquelle ledit boîtier (10) de dispositif de surveillance comprend des moyens tels que des boutons poussoirs ou des commutateurs pour interrompre manuellement lesdites première et deuxième alarmes.

7. Combinaison selon l'une quelconque des revendications 1-6, dans laquelle lesdits premier et deuxième signaux comprennent un code reconnaissable par ledit premier et troisième circuit électronique dudit dispositif de surveillance (9).

8. Combinaison selon la revendication 7, dans laquelle ledit code est généré par le cinquième circuit électronique dudit dispositif médical (1) et transmis audit premier et troisième circuit électronique dudit dispositif de surveillance (9) par ledit premier et deuxième ensembles de contacts électriques (11, 11a).

9. Combinaison selon l'une quelconque des revendications 8, dans laquelle ledit circuit électronique du dispositif médical (1) comprend moyens de blocage pour bloquer la transmission de données et énergie électrique audit dispositif médical (1) si lesdits premier et deuxième contacts électriques (11, 11a) ne sont pas en contact électrique mutuel.

10. Combinaison selon l'une quelconque des revendications précédentes, dans laquelle ledit dispositif médical (1) et ledit dispositif de surveillance (9) comprennent chacun un microprocesseur agencé dans une carte de circuit imprimé (PCB) (33, 23), lesdits microprocesseurs étant en contact électrique pour échanger des données électroniques et ledit microprocesseur de dispositif médical contrôlant l'opération dudit dispositif médical (1).

11. Combinaison selon la revendication 10, dans laquelle ledit microprocesseur de dispositif de surveillance est préprogrammé pour transmettre uniquement un ensemble spécifique d'instructions de fonctionnement audit microprocesseur de dispositif médical.

12. Combinaison selon l'une quelconque des revendications précédentes, dans laquelle ledit boîtier (10) dudit dispositif de surveillance (9) est pourvu d'un affichage (41) adapté pour afficher des informations d'une manière visuelle.

13. Procédé de surveillance de la fonction d'un dispositif médical (1) l'utilisation dans des hôpitaux et analogues et sous la forme d'un ensemble de perfusion goutte à goutte portable du type comprenant une alimentation en liquide, une chambre d'écoulement en aval de ladite alimentation en liquide pour former des gouttes de liquide et un tube flexible reliant ladite chambre d'écoulement avec une aiguille d'injection, le procédé comprenant les étapes de:
- fournir une combinaison d'un dispositif de surveillance portable (9) capable de recevoir des signaux sans fil provenant dudit dispositif médical (1), et dudit dispositif médical (1), la combinaison état selon l'une quelconque des revendications précédentes,
- amener ledit dispositif médical (1) à envoyer un premier signal sans fil à certains intervalles de temps, de préférence toutes les 30 secondes, et
- amener ledit dispositif de surveillance (9) à émettre une première alarme si ledit dispositif de surveillance (9) ne reçoit pas ledit premier signal après que ledit intervalle de temps s'est écoulé, et
- connecter électriquement ledit dispositif de surveillance (9) audit dispositif médical (1),
- programmer les fonctions dudit dispositif médical (1),
- retirer ledit dispositif de surveillance (9) du contact électrique avec ledit dispositif médical (1), et
- surveiller ledit dispositif de surveillance (9) de manière à enregistrer si une alarme est émise par ledit dispositif de surveillance (9);
**caractérisé en ce que**
ledit dispositif médical (1) et ledit dispositif de surveillance (9) sont chacun munis de contacts électriques collaboratifs (11, 11a) pour établir un contact électrique direct entre ledit dispositif de surveillance (9) et ledit dispositif médical (1) pour transmettre de l'énergie électrique et données électroniques, et dans lequel les fonctions dudit dispositif médical (1) ne sont programmables que lorsque ledit contact électrique direct est ininterrompu.
